# EUROPEAN PATENT SPECIFICATION

(11) **EP 3 003 188 B1**
(45) Date of publication and mention of the grant of the patent: **15.05.2019**
(21) Application number: 14729304.7
(22) Date of filing: 06.06.2014
(51) Int. Cl.: A61B 18/04, A61B 17/42

(54) **AN APPARATUS FOR THERMAL ABLATION**
WÄRMEABLATIONSGERÄT
APPAREIL D'ABLATION THERMALE

(30) Priority: 07.06.2013 DK 201370312; 29.04.2014 DK 201470261
(43) Date of publication of application: 13.04.2016
(73) Proprietor: Lina Medical International Operations AG, 6039 Root D4 (CH)
(72) Inventor: POULSEN, Henrik Bisgaard, DK-3550 Slangerup (DK)
(74) Representative: Inspicos P/S
(86) International application number: PCT/EP2014/061894
(87) International publication number: WO 2014/195490

(56) References cited:
- EP-A2- 0 873 723
- WO-A1-94/21203
- WO-A1-2009/149321
- US-A1- 2002 151 882
- US-A1- 2004 267 340
- US-A1- 2011 152 722

## Description

### FIELD OF THE INVENTION

The invention relates to an apparatus for thermal ablation at a site in a subject. Particularly, the invention relates to an apparatus for treating uterine disorders by effecting necrosis of a uterine endometrium by use of a distendable balloon which is inserted into the uterus.

### DESCRIPTION OF RELATED ART

Application of thermal energy is known for treating body tissues. Particularly, it is well known to effect necrosis of the endometrium e.g. by use of an expandable balloon or bladder which is filled with an inflation medium at an elevated temperature, typically about 80-90° Celsius.

In an unexpanded state, the balloon is inserted into the uterus of the subject and a hot inflation medium is displaced into the balloon which thereby expands. Close contact between the hot outer surface of the balloon and the tissue lining for which necrosis is desired is maintained typically for 8-15 minutes after which the inflation medium is drained from the balloon. The collapsed balloon can finally be removed from the subject.

In some devices, the inflation medium is preheated outside the body, and then displaced into the balloon. In another type of device, the balloon houses a heating means which can heat the inflation medium once it is inside the balloon.

The heating of the inflation medium and the displacement into and out of the balloon require monitoring by the medical practitioner. If the balloon is filled too much, too fast, or with an inflation medium which is too hot, it may have detrimental effect on the subject.

US 2002/151882 A1 discloses a device for uterine endometrial ablation. Further background prior art is disclosed in US 2004/267340 A1, WO 94/21203 A1, WO 2009/149321 A1, US 2011/152722 A1 and EP 0 873 723 A2.

### SUMMARY OF THE INVENTION

It is an object of embodiments of the invention to improve the existing devices for conducting thermal ablation and particularly to provide a more simple and reliable device which facilitates improved pressure and temperature control, improved monitoring, and reduced system costs.

It is a further object to increase the safety and to reduce the duration of thermal ablation procedures.

The invention provides an apparatus according to claim 1. The inflation means counteracts a change in pressure caused by heating of the medium - i.e. when the inflation medium is heated and thereby expands, the expansion is counteracted by the inflation means such that the result is an equal or essentially equal pressure in the bladder or at least a pressure which is kept within a specific range or a pressure which is lowered during the heating.

Thermal expansion is the tendency of matter to change volume in response to a change in temperature. Due to the heating of the inflation medium, the medium expands and the pressure therefore increases. Since the inflation means is operated in the negative direction during heating, the inflation means counteracts the pressure increase. The invention thereby prevents an unintended and early inflation of the bladder before it is inserted into the body of the treated patient.

This again facilitates insertion of the bladder e.g. into the uterus directly following the heating once the correct temperature is obtained and without firstly having to deflate a partly inflated bladder or without having to force a partly inflated bladder through the cervix.

The inflation means may counteract a change in pressure either by powered, electrical operation of the inflation means, or the change in pressure may be counteracted by the increasing pressure itself, e.g. by displacing a component of the inflation means by use of the increasing pressure itself.

According to the invention, the apparatus further comprises control means communicating with the inflation means and a pressure detection means for regulating the inflation. The inflation means and the pressure detection means operate with electrical signals and the inflation means may particularly powered by electrical power. The control means is preferably a computer control means including a CPU (computer processing unit).

The control means is configured to provide an electrical signal for operating the inflation means in the negative direction during heating of the inflation medium.

For easy insertion of the bladder, it may be an advantage to provide the apparatus to the end-user in a completely deflated state, and possibly with the bladder wrapped at least partly around the catheter so that it forms a relatively small diameter. In this case, the invention prevents that the bladder inflates during heating and thereby moves away from the intended and planned small diameter size which is suitable for insertion.

As it appears from claim 1, the following definitions apply herein. Proximal end is defined as that end where the bladder is attached, i.e. the end pointing towards the patient during treatment. The distal end is the opposite end pointing away from the patient. Likewise, the proximal direction is the direction towards the patient and distal direction is the direction away from the patient during treatment.

The pressure of the inflation medium could be detected, e.g. continuously or in discrete steps. The detection may be carried out simultaneously with the heating of the inflation medium. Based on the pressure, the inflation means is operated in the negative direction, e.g. such that the pressure is maintained essentially constant and such that distension of the bladder during heating of the inflation medium is avoided. The inflation means could be operated in the negative direction in discrete steps or continuously. In one embodiment, the pressure of the inflation medium is detected in discrete steps simultaneously with the heating of the inflation medium and the inflation means is operated in the negative direction in corresponding discrete steps, each step following a step of detecting the pressure of the inflation medium.

The control means may include an electronic circuit, e.g. having memory means with a predefined pressure range stored therein and indicating a desired pressure in the bladder during heating, and optionally also during a subsequent treatment of the patient.

The pressure range specifies at least a maximum pressure during the heating process and/or during the treatment. When reaching the maximum pressure, the inflation means is operated in the negative direction until the pressure becomes below the maximum pressure.

In one embodiment, the pressure range also specifies a minimum pressure. When the inflation means is operated, the operation is continued until the pressure in the bladder is below the minimum pressure. In one embodiment, the pressure range can be re-specified, e.g. to match specific national requirements with regard to pressure or temperature etc., e.g. to match specific external pressures e.g. depending on geographical location.

Since the increase in pressure is a result of thermal expansion, a more exact and faster reacting system may be obtained by controlling the inflation means based on the temperature of the inflation medium. For that purpose, the apparatus may comprise temperature detection means arranged to detect the temperature of the inflation medium, and the control means may be configured to operate the inflation means in the negative direction in response to a detected increase in temperature.

The control means may be configured to generate an error signal if the pressure of the inflation medium does not increase during heating. In one example, the control means is configured to generate the error signal if the inflation means is not operated in the negative direction during heating of the medium. This may be a simple and yet effective way of detecting errors in the system, particularly to detect leakage of the inflation medium. Alternative or additional leak detection means may comprise means for surveillance of a pressure signal for detection of the pressure being maintained above a threshold value. The threshold value could be stored in the aforementioned memory means and it could be a value which can be redefined during manufacturing or by the end user.

The control means may further be configured to generate an inflation signal indicating that the bladder is inflated. The inflation signal may particular be useful once the bladder is inserted into the uterus and therefore can't be observed visually. The inflation signal may indicate when the bladder can be removed and when it can't be removed from the uterus.

The control means may further comprise a timer and memory means. The memory means may include information specifying a pre-defined sequence and a pre-defined period of time. In this embodiment, the control means may be configured to effect the pre-defined sequence which may particularly include the following steps:
a) Firstly, the inflation medium is heated with simultaneous pressure correction by operation of the deflation means as described above; The first step is typically carried out before the bladder is inserted into the uterus;
b) When the inflation medium is heated, the control means may provide a ready signal indicating that the bladder may now be inserted in the uterus and that the ablation can therefore begin;
c) Secondly, the inflation means is operated in the positive direction to inflate the bladder; before this second step, the control means may wait for an activation signal to be given by the user. For this purpose, the apparatus may include a button or similar control for starting the process when the bladder is inserted into the uterus.
d) Thirdly, the pressure in the bladder is maintained during the pre-defined period of time;
e) Fourthly, the inflation means is operated in the negative direction to deflate the bladder; and
f) Finally, a finish signal indicating deflation of the bladder is transmitted and the bladder can be removed from the uterus.

The process may include further steps of repeated deflation and inflation of the bladder. This is carried out between the above mentioned step c) and d) such that the bladder is repeatedly emptied and filled with the inflation medium during the pre-defined period of time. For each of these cycles, the bladder may be kept in the inflated state for a period in the range of 10-50 percent of the pre-defined period of time.

In the claimed invention, the apparatus comprises a storage chamber containing an amount of the inflation medium, and the inflation means pumps the inflation medium from that storage chamber into the bladder.

The storage chamber may contain a larger amount of the inflation medium than what is necessary for inflating the bladder. In this embodiment, the heating means may be configured to heat the entire amount of the inflation medium, and the repeated deflation and inflation of the bladder may cause mixing of that portion of the inflation medium which is in the bladder with that portion of the inflation medium which is in the storage chamber. As a result of the repeated deflation and inflation of the bladder, the bladder may become reheated several times during the treatment, and a more constant high temperature of the surface of the bladder can be obtained.

The storage chamber may be formed by the inflation means itself - i.e. the storage chamber may form a chamber of a pump which is used for inflating the bladder.

The inflation means may particularly include power driven means and it may constitute a power driven pump, e.g. a displacement pump, or a centrifugal pump. The pump may e.g. form the structure of a syringe or the structure of a peristaltic pump etc. The apparatus may further comprise motor control means configured to determine a power consumption of the inflation means. The previously mentioned step f) of transmitting a finish signal may be triggered by detection of increased power consumption which will typically occur when the power driven pump reaches an end-stop.

The use of a power driven syringe facilitates in a simple manner, an exact displacement of the inflation medium into the bladder and thus controlled expansion of the bladder.

The power driven means may work on the cylinder or on the piston to move that element relative to the other element of the syringe structure. The syringe structure also effectively forms the aforementioned storage chamber whereby the inflation means itself forms the storage chamber.

Particularly, the combination between power driven means and a syringe structure enables precise dosing of the inflation medium into and out of the bladder by use of very simple and cheap motors. This is particularly important relative to counteracting the change in pressure caused by thermal expansion of the inflation medium during heating since it allows a precise adjustment of the pressure and thus avoidance of unintended inflation during heating.

Relative displacement of the cylinder and piston may e.g. be effected by a worm shaft etc. or it may generally be based on a threaded engagement between a driven and a driving element, e.g. between a nut which is rotated by the motor and therefore constitutes the driving element and a threaded piston or threaded element connected to the piston and which thereby constitutes the driven element.

The power driven means used for driving the inflation means could particularly be constituted by a rotary motor of the kind including a rotor and a stator, e.g. a DC motor. Alternatively or additionally, the power driven means may include electromechanical actuation means, e.g. in the form of a solenoid operating e.g. to move a piston and cylinder relative to each other.

The apparatus may particularly be independently powered. Herein, independent powering means that the device contains a local source of electrical energy, in the following simply referred to as *a battery.*

By the term *"battery"* is herein meant a number of cells, e.g. 1, 2, 3, 4 or more cells, each capable of delivering electrical power. Particularly, the battery may comprise at least one electrochemical cell and/or at least one capacitor.

The battery may typically deliver between 3 and 20 volt and have about 500-2600 mAH of capacity. It may be for disposable, one time usage or it may be rechargeable for multiple usages.

Particularly, it is an object to make a completely independent, single piece device, e.g. for single use. Typically, however, batteries should be disposed in containers specifically for receiving batteries, and typically, instruments which may have been contaminated with biological material, such as blood and tissue, should be disposed in other containers specifically for that purpose. It may therefore be an advantage if the device comprises a detachable independent powering means designed for intended destruction by which the battery, capacitor, or similar power source becomes detached from the chassis such that reassembly becomes difficult or impossible.

To make a completely independent device, not only power but also all other necessary elements may be included in a single piece device. Accordingly, the apparatus may be formed by a single entity or component which comprises all the claimed features including the inflation medium and the inflation means such that a separate supply of the inflation medium becomes unnecessary.

The bladder may be pre-shaped e.g. to approximate the bicornual shape of the uterus. It may be manufactured from a bio-compatible, non-allergenic material, and it may come in different sizes, e.g. in two pre-shaped sizes; one for nulliparous uteri and one for parous uteri. The bladder may also have completely different shapes for non-endometrial balloon ablation, e.g. for prostatic ablation.

The bladder could be made from an elastically deformable rubber, silicone or latex material. In one embodiment, the bladder comprises at least a first and a second balloon positioned one within the other to increase safety if one balloon should be ruptured.

In one particular embodiment, the bladder comprises a first and a second balloon, one within the other, and the inflation medium is injectable between the two balloons, i.e. the space between the first and second balloon forms a reservoir for the heated inflation medium while the inner balloon could be filed with an alternative inflation medium, e.g. in an unheated state.

In this embodiment, the inner balloon may e.g. be expanded by air. Since it is only the space between the first and second balloon which is filled with the heated inflation medium, the amount of heated inflation medium which is necessary for a treatment can be reduced whereby the thermal capacity of the system is reduced. This reduces also the necessary thermal energy for bringing the inflation medium to the requested temperature and the time it takes to heat the inflation medium. As a further advantage, the inflation medium cools down faster and the risk of unintended burns can be reduced.

The heating means may e.g. be incorporated in, or it may form part of the inflation means. Particularly, the heating means may form part of the previously described cylinder or piston. In this way, relative movement between the cylinder and piston causes also relative movement between the heating means and one of the cylinder and piston. This may increase the thermal convection and facilitate a more homogeneous temperature of the inflation medium.

The inflation medium may particularly be heated to a temperature above 100°C and more particularly to a temperature above 130°C such as to a temperature in the range of 120-150°C or to a temperature in the range of 120-160°C. To reach this temperature, the inflation medium may particularly be a liquid with a boiling point above 150°C and preferably even above 200°C. The inflation medium may particularly be glycerol, e.g. C₃H₈O₃.

Further, the bladder may desirably be made from a material which resists temperatures above 150°C, or above 200°C, and desirable be made from a material which exposes at most 5 per cent change in module of elasticity during a temperature increase from 20°C to 150°C, where the module of elasticity is defined as a tendency of the material to be deformed elastically, i.e., non-permanently, when a force is applied to it.

This relatively high temperature may reduce the duration of the treatment but may introduce a risk of damaging the cervix and vaginal tissue lining. To prevent such damages, the entire catheter, or at least an insertable part thereof, or at least the proximal end, may preferably be made such that the thermal spreading from the inner surface of the catheter to the outer surface of the catheter is low.

The *"insertable part"* is herein defined as that part of the catheter which, during use of the device, is inserted into the body of the treated subject, i.e. e.g. into the vaginal canal or the cervical canal. The *"proximal end"* is herein defined as less than half of the length of the catheter at that end where the bladder is attached to the catheter, i.e. from the bladder and at most half way down, e.g. 1/3, or 1/4 of the way towards the proximal end of the catheter.

In one embodiment, the thermal conductivity of the insertable part or of the proximal end is lower than the remaining portion of the catheter.

In one embodiment, the entire catheter, the insertable part, or the proximal end has a lower thermal conductivity than the bladder.

In one embodiment, the entire catheter, the insertable part, or the proximal end has first and second coaxial elements extending about a conduit, the first and second elements have different thermal conductivity. Due to the different thermal conductivity, the propagation of thermal energy through the wall of the catheter may be reduced.

Particularly, one of the elements may have a thermal conductivity less than one tenth of the thermal conductivity of the bladder and/or less than one tenth of the thermal conductivity of the other element of the catheter. One element could e.g. be made from steel, e.g. from titanium or stainless steel and the other element could be made from plastic. Preferably the outer element could be made from plastic while the inner element is made from steel.

In one embodiment, the catheter has a layered structure with 3 layers being an inner layer, an intermediate layer and an outer layer.

The inner layer, e.g. of steel, has a relatively small layer thickness. This layer forms structural rigidity and forms the conduit for the inflation medium. The intermediate layer is for thermal isolation and contains a large amount of gas or air. Typically, this material is a foam material, e.g. polyimide. The intermediate layer is in the range of 3-20 times thicker than the inner layer. The outer layer is for encapsulating the intermediate layer and it has a lower surface friction than the intermediate layer. Typically, the intermediate layer is in the range of 1-10 times thicker than the outer layer.

In one particular embodiment, the catheter has an outer diameter being 5.4 mm. i.e. the outer layer forms a tube with a diameter of 5.4 mm. The intermediate layer forms a tube with a diameter of 5.0 mm. The inner layer forms a tube with a diameter of 2.5 mm, and the conduit formed within the inner layer has a diameter of 2.1 mm.

Fibre composite materials typically have a low thermal conductivity. To prevent excessive temperatures on the outer surface of the catheter, the entire catheter, the insertable part, or the proximal end of the catheter could be made from a fibre composite material, e.g. a glass fibre or carbon fibre reinforced polymer material.

The entire catheter, the insertable part, or the proximal end could be covered at least partly with a surface layer of a bio-compatible material, e.g. with a hydrophilic coating, coating of Poly-tetra-fluoro Ethylene (PTFE), or simply coated with a layer of hydrogel. PTFE may provide an additional advantage since it has a very low thermal conductivity and it may therefore prevent high temperatures on the outer surface of the catheter.

To further prevent damages to the cervix and the vaginal lining, the inflation means may inflate the bladder while the inflation medium is heated. In that way, the vaginal lining is contact with the bladder while the temperature increases, and the duration in which the vaginal lining is in contact with the maximum temperature can be reduced.

For emergency purpose, e.g. if the inflation means fails, the device may comprise an emergency release structure allowing inflation medium to be drained from the bladder and thus allow collapsing of the bladder without operation of the inflation means.

The apparatus may e.g. comprise a disposal storage for disposal of the inflation medium which is drained from the bladder during an emergency procedure.

To avoid spillage of the inflation medium and to avoid potential scalds caused by the hot inflation medium, the apparatus may comprise a liquid absorbing material arranged to receive the inflation medium which is drained from the bladder. The liquid absorbing material may e.g. include a hydrophilic material, e.g. including polyvinylpyrolidone (PVP) or other materials well known for their liquid absorbing properties, and it may e.g. be included in the disposal storage.

The emergency release structure may comprise an exit which is sealed by an emergency valve structure, e.g. a manually operable valve.

To prevent reuse of the device, the emergency valve structure may be operable from a closed position to an open position, but not reversible to the closed position. I.e. once opened, the inflation medium will drain out of the bladder and the emergency valve will remain open whereby the apparatus becomes unsuitable for further use.

The device may comprise separate sensors for sensing temperature and pressure. The device may also comprise several sensors capable of sensing temperature and/or several sensors capable of sensing pressure, and control logic capable of reading several pressure and/or temperature signals from the sensors and to determine a fault situation in case the difference between the signals from two identical sensors is above a limit value.

In one embodiment, the control means is configured for a fully automatic treatment. For this purpose, the control means may be configured to execute a sequence, e.g. comprising the following steps:
1. The user initiates the treatment by turning on the device. By this activity, the control means initiates heating and the pressure is compensated by movement of the inflation means in the negative direction.
2. The bladder is inserted to the site of operation, e.g. into a body cavity such as the uterus for endometrial thermal ablation.
3. When the bladder is in correct position, the user indicates that the apparatus is in position by pressing a start button. By this activity, the control means will control the inflation means to cause inflation of the bladder. At the same time, a timer could started.
4. After a predetermined duration, the control means initiates circulation of the inflation means, e.g. by emptying and re-filling the bladder a few times, e.g. 2, 3 or 4 times.
5. After a predetermined duration, the control means controls the inflation means to operate in the negative direction and the bladder is emptied. When the bladder is empty, the user is notified that the treatment is finalized and the bladder can be removed from the uterus.

Since the control means is fully automatic, the entire treatment is carried out essentially without intervention from the user and the risk of faults is reduced.

To ensure only one-time usage of the device and disposal of the device after use, the control means may be configured to only allow one single treatment after which the apparatus stops working.

To prevent damaging the bladder during insertion of the catheter into a body cavity such as uterus, the device may comprise a transition body forming a distal termination of the catheter and located inside the bladder. The transition body is softer than the catheter such that it easily deforms and flattens out when reaching the bottom wall of the body cavity into which the bladder is inserted.

Herein, softer is defined as *"obtaining a certain degree of elastic deformation by a lower pressure".*

To seal the body cavity during the thermal ablation procedure, e.g. to seal uterus, the device may comprise a sealing member forming a protrusion on an outer surface of the catheter. The sealing member may e.g. be made from a soft, i.e. easily elastically deformable, polymer material, e.g. a hydrophilic material, e.g. a material containing acrylamide, polyvinylpyrolidone or other hydrophilic materials prepared such that they swell.

Particularly, the sealing member may extend circumferentially on an outer surface of the catheter.

In one embodiment, the sealing member is slidable axially along the outer surface of the catheter to thereby enable positioning of the sealing member at a distance from the tip of the catheter which corresponds to the depth of uterus and cervix of the person which is treated.

In one embodiment, the sealing member is made to expand upon contact with the inflation medium contained in the bladder, or upon contact with body liquids. In that way, leakage of the hot and potentially damaging inflation medium e.g. from uterus to the cervical or vaginal canal, e.g. if the bladder is ruptured, can be prevented. The sealing member may form one or more stripes circumferentially about an outer surface of the catheter, e.g. made by coating the outer surface of the catheter with a hydrophilic materiel.

When the catheter is inserted e.g. through cervix, the hydrophilic coating may swell upon absorption of body inflation mediums, but particularly, it may swell upon leakage of the inflation medium from the bladder e.g. by absorbing that inflation medium, if the bladder is ruptured. When the sealing body is swelled, it may provide a seal between the cervical canal and the catheter.

The catheter, the bladder, the inflation means, the heating means, and the control means could be joined inseparably to form integral parts of a mobile unit. A battery powering the inflation means and the heating means could also form an integral, inseparable part, or it could be releasable from the mobile unit.

By mobile unit is herein meant a unit which does not need external power, i.e. it is powered by a battery or similar internally contained power source and it has a size, shape, and weight enabling it to be manipulated as a one-piece device and by hand.

The mobile unit may, in one end form a handle suitable for manipulation of the unit by hand, and the catheter and bladder may form an opposite end of the mobile unit.

In an example not according to the invention, there is provided a method of operating the apparatus of the kind described until now. Particularly, the method may be used for testing the apparatus, and it comprises the steps of heating the inflation medium and evaluating the pressure during

heating. The pressure could be evaluated e.g. by measuring the temperature of the inflation medium and/or measuring the pressure relative to an external pressure around the apparatus.

The method may further comprise the step of operating the inflation means in the negative direction during heating of the inflation medium.

### BRIEF DESCRIPTION OF THE DRAWINGS

Figs. 1 and 2 are perspective views of an assembled device according to the invention;
Fig. 3 is a side view, in cross section, of a device according to the invention;
Fig. 4 illustrates details of the piston;
Fig. 5 illustrates details of the catheter in a cross sectional view;
Fig. 6 illustrates details of the chamber,
Figs. 7 and 8 illustrate further details of the chamber,
Figs. 9-11 illustrate different embodiments of a transition body at the proximal end of the catheter inside the bladder;
Figs. 12-16 illustrate different embodiments of stop and sealing members, and
Figs. 17-20 illustrate details of one embodiment of the emergency valve.

### DETAILED DESCRIPTION OF AN EMBODIMENT

Further scope of applicability of the present invention will become apparent from the following detailed description and specific examples. However, it should be understood that the detailed description and specific examples, while indicating preferred embodiments of the invention, are given by way of illustration only, since various changes and modifications within the scope of the invention will become apparent to those skilled in the art from this detailed description.

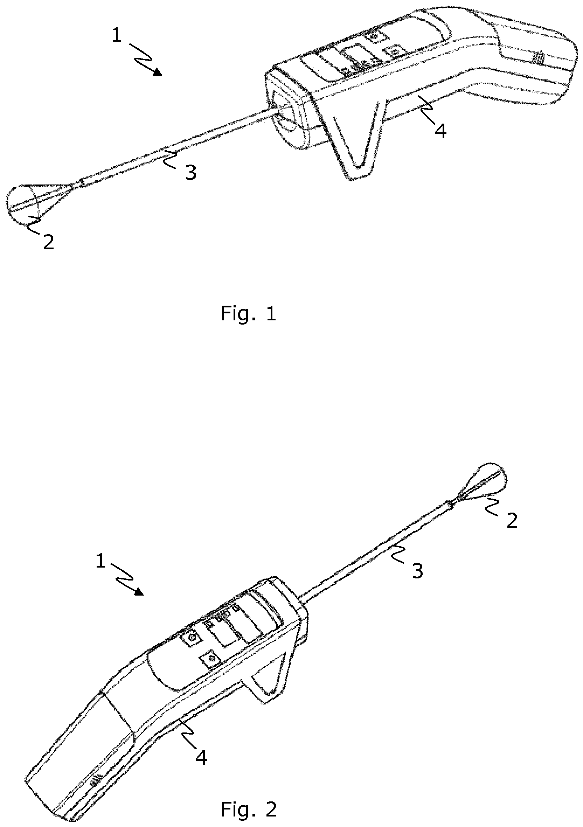

Figs. 1 and 2 illustrate a device 1 for effecting necrosis of the endometrium. The device comprises an inflation medium contained in a reservoir which is constituted by an expandable bladder 2 which is connected by an elongated catheter 3 to a displacement chamber (not shown) housed within the casing 4. The displacement chamber forms part of the inflation means.

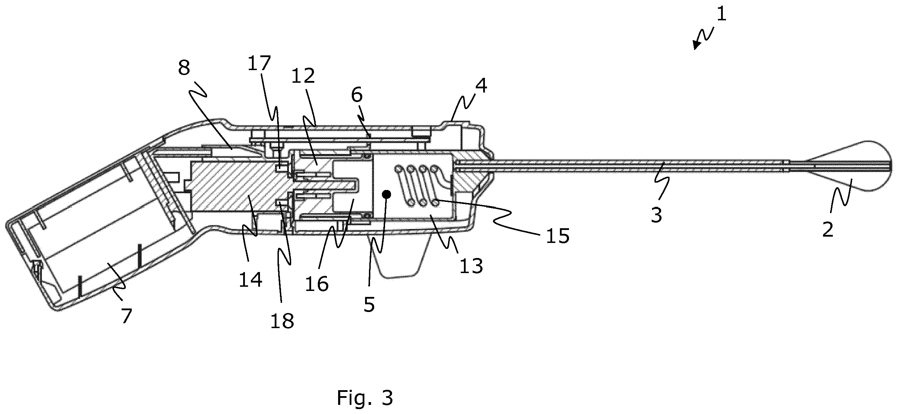

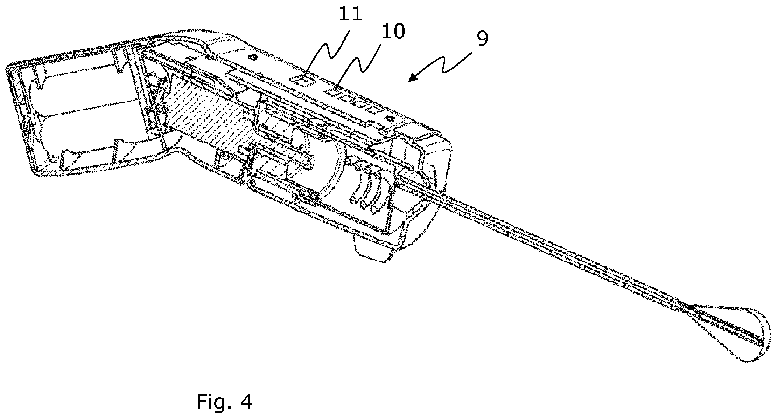

The casing is illustrated in a cross sectional view in Fig. 3. The casing houses the displacement chamber 5, a control means 6, a battery 7, a disposal storage 8, and a user interface 9 (Cf. Fig. 4) including buttons 10 for controlling operation of the device and a display 11 for monitoring the temperature and/or the duration of the treatment.

The displacement chamber is constituted by a syringe structure including a piston 12 movable in a cylinder 13 by an electrical motor 14 - in this case a DC servo motor or step-motor.

The control system may particularly provide a fully automatic system managing the entire treatment, i.e. the heating of the inflation medium, the expansion of the bladder, the duration in which the bladder is expanded and the collapsing of the bladder once the treatment is finished.

The control system may be integrated in a printed circuit board (PCB) which includes memory, a computer processing unit, and a program executable in the processing unit and configured to make the control system communicate with the heating means, the motor, and/or with the sensors to carry out the processes of:
- heating the inflation medium until a predetermined temperature is achieved;
- operating the motor to control the pressure in the bladder and to inflate and deflate the bladder;
- counting a duration by a timer;
- notifying the user when the treatment is finished.

The control system may have storage means in which all data related to the treatment is stored. The control system may further have communication means adapted to provide documentation including data describing a treatment, e.g. the temperature, the duration, the pressure of the inflation medium and/or other data relevant for evaluating the treatment.

The heating means 15 is attached to, and extends inside the cylinder 13. The piston forms a cavity 16 shaped and dimensioned to receive the heating means 15.

When the piston is moved in the cylinder, the heating means becomes received in the cavity and the inflation medium therefore becomes displaced or "stirred" in the chamber in the vicinity of the heating means 15. This increases the thermal convection and provides a more equal temperature in the inflation medium.

The device further comprises a sensor 17 capable of sensing pressure and a sensor 18 capable of sensing temperature of the inflation medium in the bladder. The sensors communicate with the control means 6.

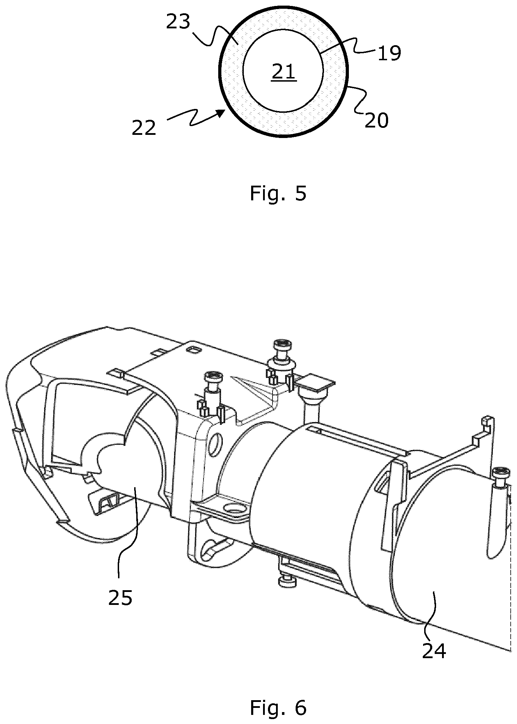

Fig. 5 illustrates details of the catheter in a cross sectional view. The catheter comprises first and second coaxial elements 19, 20 extending about a conduit 21. The two elements are made from different materials and have different thermal conductivity to thereby reduce thermal spreading from the conduit to the outer surface 22 of the catheter. Between the coaxial elements 19, 20, the device may comprise a third element 23 having very low thermal conductivity. In one embodiment, the coaxial elements 19, 20 are in direct contact without the third element.

Fig. 6 illustrates in a perspective view, the displacement chamber 24 and the motor 25 which constitutes the power driven means.

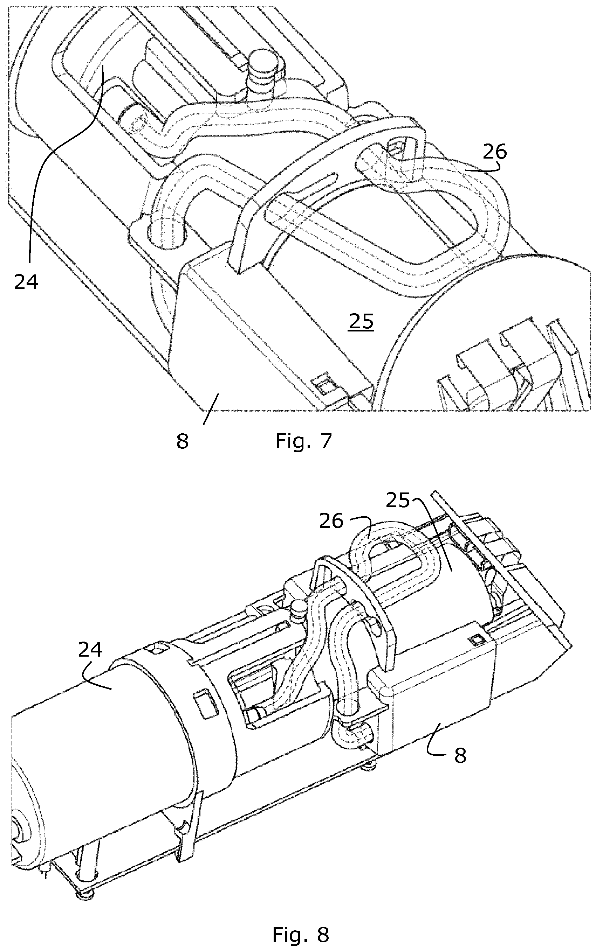

Figs. 7 and 8 illustrate further details of the chamber 24. In this view, it is illustrated that the chamber comprises an emergency release structure 26 constituted by a rubber tube. The emergency release structure is in fluid communication with the disposal storage 8 which contains a liquid absorbing material. A valve 27 controls the drainage of inflation medium into the body 8. The emergency release structure is operated via the valve e.g. if the power driven means fails, e.g. when the battery is empty or in case of faults. In this embodiment of the emergency valve 27, the valve forms a passage for the rubber tube, and the passage has two dimensions. When the rubber tube is in one part of the passage, a small dimension squeezes the rubber tube and thereby prevents a fluid flow. When the rubber tube is in another part of the passage, a large dimension allows the rubber tube to open and thereby enables a fluid flow. The rubber tube may be configured to prevent permanent deformation in the squeezed state.

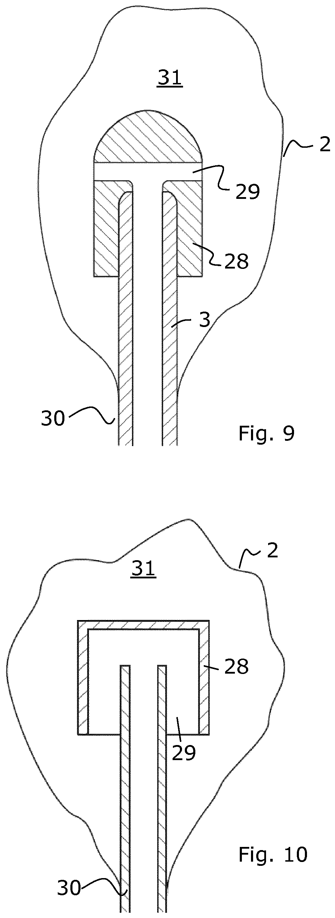

Figs. 9-11 illustrate different embodiments of a transition between the elongated catheter 3 and the expandable bladder 2. The transition includes a transition body 28 of a very soft and resilient rubber, latex, silicone or similar soft material. The transition body is attached to the proximal end of the catheter 3 or it is formed by the proximal end of the catheter 3. During insertion of the bladder into a body cavity, e.g. the uterus, the transition body may come into contact with a rear wall of the body cavity whereby the user can feel that the full depth of the body cavity has been reached. Upon contact with the rear wall of the body cavity, the transition body is deformed and thereby protects the wall of the body cavity from damages and it protects the bladder from being ruptured by a sharp tip of the catheter.

The transition body may include electronic sensing means configured to determine a distance to the rear wall of the body cavity or configured to determine impact between the transition body and the rear wall of the body cavity.

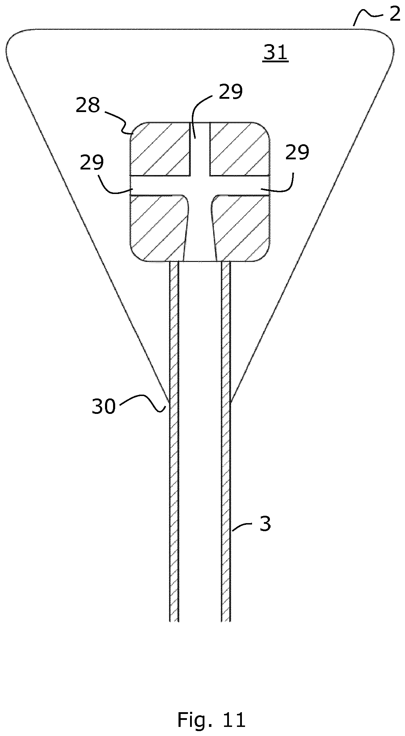

The transition body forms openings 29, e.g. sideways as illustrated in Fig. 9 or rearwards as illustrated in Fig. 10, or upwards as illustrated in Fig. 11. The openings allow the inflation medium to flow from the catheter into the bladder 2. Other softly rounded, bulbous shapes of the transition body may be used. The bladder 2 is adhesively attached to the outer wall 30 of the catheter 3 such that the transition body becomes included in the space 31 inside the bladder.

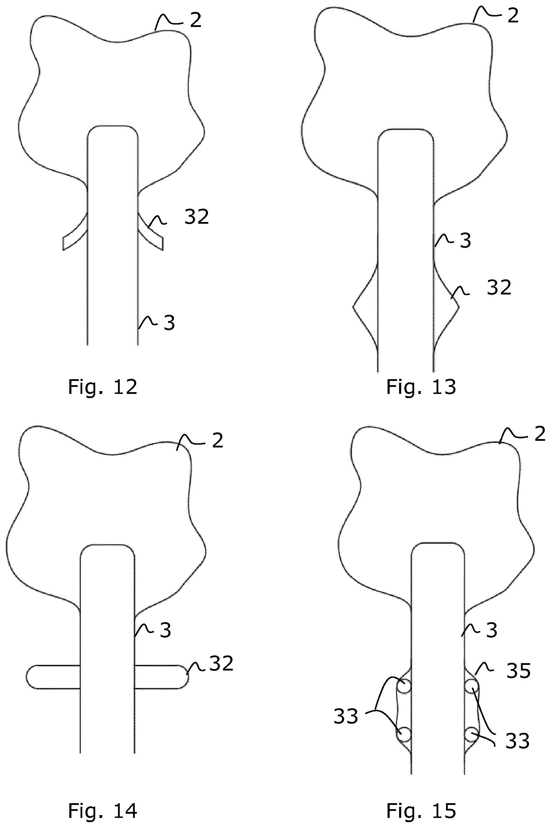

Figs. 12-15 illustrate different embodiments of stop and sealing members 32 for use of the device for endometrial ablation. The stop and sealing members cooperate with the cervix to provide a sealed passage of the catheter and bladder into the uterus. The embodiment illustrated in Fig. 15 includes two swellable bodies 33 located between an outer surface of the catheter and a sheath 35. When swelling, the swellable bodies presses the sheath against a surface of cervix and thereby seals the passage into uterus. The swellable bodies may e.g. be of a hydrophilic material.

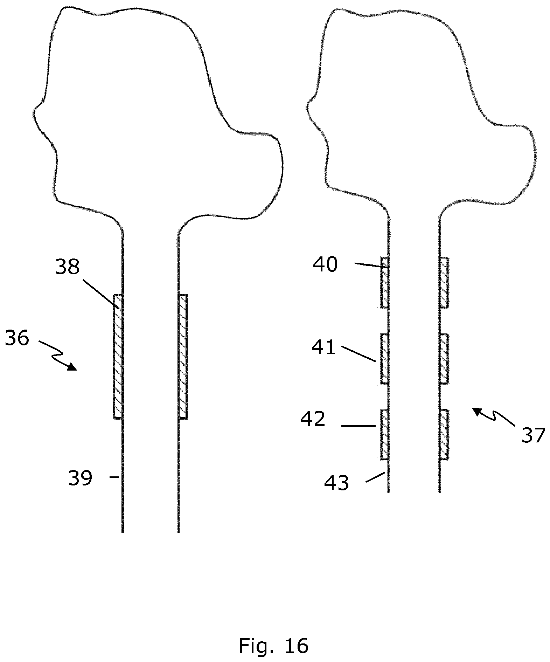

Fig. 16 illustrates two different catheters 36, 37. The catheter 36 has a single hydrophilic surface layer 38 provided as a coating about the body 39. The catheter 37 comprises three hydrophilic surface layers 40, 41, 42.

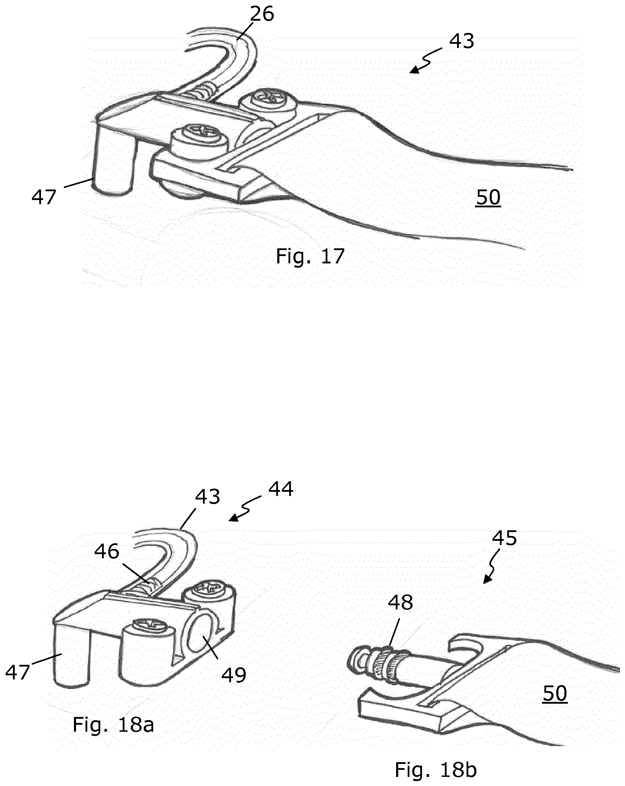

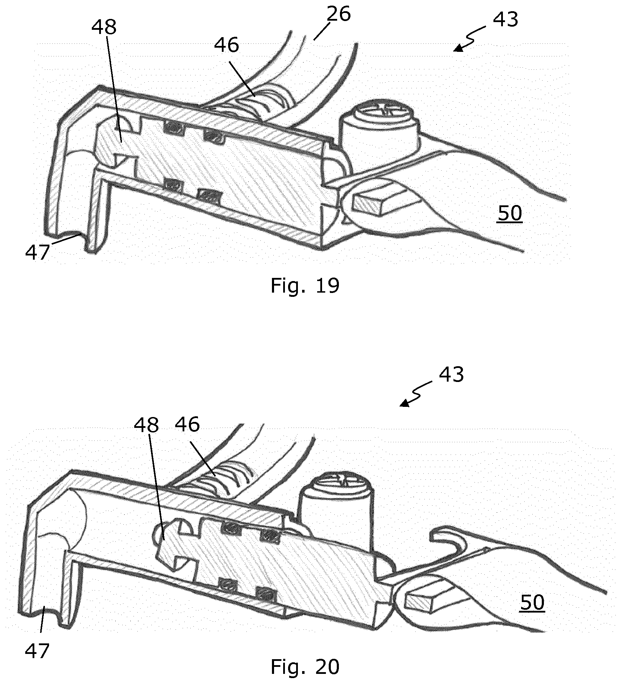

Figs. 17-20 illustrate details of another embodiment of the emergency valve 43 for the emergency exit 26, the exit and valve form an emergency release structure according to the invention.

The emergency valve controls a flow of the inflation medium into the disposal storage 8 or simply out of the apparatus. The emergency valve connects to the rubber hose 26 and comprises first and second valve parts 44, 45. The first valve part 44 receives the inflation medium via the inlet 46 and delivers the inflation medium to the outside or to the disposal storage via the outlet 47. The second valve part 45 comprises a plug element 48 insertable into the opening 49 in the first valve part 44 and thereby blocks the passage between the inlet 46 and the outlet 47. The second valve part is connected to or forms a pull tab 50 which can be reached on the outer surface of the apparatus. The user thereby operates the emergency release structure by pulling the pull tab whereby the first and second valve parts separate and the inflation medium drains out of the apparatus or into the disposal storage. As it appears from the description and drawings, the emergency release structure is completely independent on electrical power, i.e. completely non-electrically operated and activation of the emergency release structure prevents further use of the apparatus.

## Claims

1. An apparatus (1) for treating uterine disorders by effecting necrosis of a uterine endometrium, the apparatus comprising:
• a catheter (3) having a proximal end and a distal end;
• a bladder (2) attached to the proximal end for insertion into uterus and being distendable upon introduction of an inflation medium;
• a storage chamber (13) containing an amount of the inflation medium;
• inflation means (12,13,14) connected to said distal end and being operative in a positive direction introducing the inflation medium from that storage chamber into the bladder and thereby distending the bladder and in a negative direction for withdrawing the inflation medium from the bladder and thereby deflating the bladder; and
• heating means (15) configured to heat the inflation medium in the storage chamber before insertion of the bladder into the body of a subject to a temperature sufficient to effect tissue necrosis;
wherein the apparatus is configured for simultaneous operation of the heating means and the inflation means and wherein the inflation means is configured to counteract a change in pressure in the bladder caused by thermal expansion of the inflation medium during heating in the storage chamber,
further comprising electronic control means (6) communicating with the inflation means (12, 13, 14) and a pressure detection means for regulating the inflation, and
wherein the control means is configured to provide an electrical signal for operating the inflation means to counteract a change in pressure in the bladder caused by thermal expansion of the inflation medium during heating in the storage chamber before insertion of the bladder into the body of a subject.

2. An apparatus according to claim 1, further comprising pressure detection means (17) arranged to detect a pressure of the inflation medium, and wherein the control means is configured to operate the inflation means in the negative direction in response to a detected increase in pressure of the inflation medium.

3. An apparatus according to any of the preceding claims, further comprising temperature detection means (18) arranged to detect the temperature of the inflation medium, and wherein the control means is configured to operate the inflation means in the negative direction in response to a detected increase in temperature.

4. An apparatus according to any of the preceding claims, where the control means is configured to generate an error signal if the inflation means is not-operated in the negative direction during heating.

5. An apparatus according to any of the preceding claims, where the control means is configured to generate an error signal if the pressure of the inflation medium does not increase during heating to thereby provide leak detection.

6. An apparatus according to any of the preceding claims, where the control means comprises memory means, the memory means including information specifying a pre-defined sequence causing thermal ablation of a patient, the sequence including the step of counteracting the change in pressure caused by thermal expansion of the inflation medium during heating.

7. An apparatus according to claim 6, where the pre-defined sequence comprises the steps of:
a) operating the inflation means in the positive direction to inflate the bladder;
b) maintaining the pressure in the bladder during the pre-defined period of time;
c) operating the inflation means in the negative direction to deflate the bladder; and
d) transmitting a finish signal indicating deflation of the bladder.

8. An apparatus according to claim 7, comprising motor control means configured to determine a power consumption of the inflation means and where the finish signal is transmitted in response to detection of increased power consumption.

9. An apparatus according to any of the preceding claims, where the inflation means pumps the inflation medium from that storage chamber into the bladder.

10. An apparatus according to claim 9, where the storage chamber contains a larger amount of the inflation medium than what is necessary for inflating the bladder.

11. An apparatus according to claim 10, where the heating means is configured to heat the entire amount of the inflation medium contained in the storage chamber and where the inflation means is adapted to effect repeated deflation and inflation of the bladder to thereby mix a portion of the inflation medium which is in the bladder with a portion of the inflation medium which is in the storage chamber.

12. An apparatus according to any of the preceding claims, where the inflation means comprises a displacement chamber (12, 13) having a variable volume, the chamber comprises a syringe structure including a piston which is movable in a cylinder by power driven means for varying the volume of the displacement chamber.

13. An apparatus according to any of the preceding claims, where the inflation means is driven by power driven means (14) powered by a battery (7).

## Patentansprüche

1. Vorrichtung (1) zur Behandlung von Gebärmutterstörungen durch Bewirken der Nekrose eines Gebärmutterendometriums, wobei die Vorrichtung umfasst:
• einen Katheter (3), der ein proximales Ende und ein distales Ende aufweist;
• eine Blase (2), die an dem proximalen Ende zum Einführen in die Gebärmutter befestigt ist und bei Einleitung eines Aufblasmediums ausdehnbar ist;
• eine Speicherkammer (13), die eine Menge des Aufblasmediums enthält;
• eine Aufblaseinrichtung (12, 13, 14), die mit dem distalen Ende verbunden ist und in einer positiven Richtung betreibbar ist, wobei sie das Aufblasmedium aus dieser Speicherkammer in die Blase einleitet und dadurch die Blase ausdehnt, und in einer negativen Richtung, um das Aufblasmedium aus der Blase zurückzuziehen und dadurch die Blase zu entleeren; und
• eine Heizeinrichtung (15), die konfiguriert ist, um das Aufblasmedium in der Speicherkammer vor dem Einführen der Blase in den Körper einer Person auf eine Temperatur zu erwärmen, die ausreicht, um eine Gewebenekrose zu bewirken;
wobei die Vorrichtung für den gleichzeitigen Betrieb der Heizeinrichtung und der Aufblaseinrichtung konfiguriert ist und wobei die Aufblaseinrichtung konfiguriert ist, um einer Druckänderung in der Blase entgegenzuwirken, die durch die thermische Ausdehnung des Aufblasmediums während des Erwärmens in der Speicherkammer verursacht wird,
ferner umfassend eine elektronische Steuereinrichtung (6), die mit der Aufblaseinrichtung (12, 13, 14) in Verbindung steht, und eine Druckerfassungseinrichtung zum Regeln des Aufblasens, und
wobei die Steuereinrichtung konfiguriert ist, um ein elektrisches Signal zum Betreiben der Aufblaseinrichtung bereitzustellen, um einer Druckänderung in der Blase entgegenzuwirken, die durch die thermische Ausdehnung des Aufblasmediums während des Erwärmens in der Speicherkammer vor dem Einführen der Blase in den Körper einer Person verursacht wird.

2. Vorrichtung nach Anspruch 1, ferner umfassend eine Druckerfassungseinrichtung (17), die eingerichtet ist, um einen Druck des Aufblasmediums zu erfassen, und wobei die Steuereinrichtung konfiguriert ist, um die Aufblaseinrichtung in der negativen Richtung als Reaktion auf einen erfassten Anstieg des Drucks von dem Aufblasmedium zu betreiben.

3. Vorrichtung nach einem der vorhergehenden Ansprüche, ferner umfassend eine Temperaturerfassungseinrichtung (18), die eingerichtet ist, um die Temperatur des Aufblasmediums zu erfassen, und wobei die Steuereinrichtung konfiguriert ist, um die Aufblaseinrichtung in der negativen Richtung als Reaktion auf einen erfassten Anstieg der Temperatur zu betreiben.

4. Vorrichtung nach einem der vorhergehenden Ansprüche, wobei die Steuereinrichtung konfiguriert ist, um ein Fehlersignal zu erzeugen, wenn die Aufblaseinrichtung während des Erwärmens nicht in die negative Richtung betrieben wird.

5. Vorrichtung nach einem der vorhergehenden Ansprüche, wobei die Steuereinrichtung konfiguriert ist, um ein Fehlersignal zu erzeugen, wenn der Druck des Aufblasmediums während des Erwärmens nicht ansteigt, um dadurch eine Lecksuche vorzusehen.

6. Vorrichtung nach einem der vorhergehenden Ansprüche, wobei die Steuereinrichtung eine Speichereinrichtung aufweist, wobei die Speichereinrichtung Informationen beinhaltet, die eine vordefinierte Reihenfolge spezifizieren, welche eine Wärmeablation eines Patienten verursacht, wobei die Reihenfolge den Schritt beinhaltet, der Änderung des Drucks entgegenzuwirken, welche durch die thermische Ausdehnung des Aufblasmediums während des Erwärmens verursacht wird.

7. Vorrichtung nach Anspruch 6, wobei die vordefinierte Reihenfolge die folgenden Schritte umfasst:
a) Betreiben des Aufblasmittels in der positiven Richtung zum Aufblasen der Blase;
b) Aufrechterhalten des Drucks in der Blase während der vordefinierten Zeitspanne;
c) Betreiben des Aufblasmittels in der negativen Richtung zur Entleerung der Blase; und
d) Übertragen eines Beendigungssignals, das eine Entleerung der Blase anzeigt.

8. Vorrichtung nach Anspruch 7, umfassend eine Motorsteuereinrichtung, die konfiguriert ist, um eine Leistungsaufnahme der Aufblaseinrichtung zu bestimmen, und wobei das Beendigungssignal als Reaktion auf das Erfassen einer erhöhten Leistungsaufnahme übertragen wird.

9. Vorrichtung nach einem der vorhergehenden Ansprüche, wobei die Aufblaseinrichtung das Aufblasmedium aus dieser Speicherkammer in die Blase pumpt.

10. Vorrichtung nach Anspruch 9, wobei die Speicherkammer eine größere Menge des Aufblasmediums enthält, als diejenige, die zum Aufblasen der Blase erforderlich ist.

11. Vorrichtung nach Anspruch 10, wobei die Heizeinrichtung konfiguriert ist, um die gesamte Menge des in der Speicherkammer enthaltenen Aufblasmediums zu erwärmen, und wobei die Aufblaseinrichtung eingerichtet ist, um ein wiederholtes Entleeren und Aufblasen der Blase zu bewirken, um dadurch einen Teil des Aufblasmediums, das sich in der Blase befindet, mit einem Teil des Aufblasmediums, der sich in der Speicherkammer befindet, zu vermischen.

12. Vorrichtung nach einem der vorhergehenden Ansprüche, wobei die Aufblaseinrichtung eine Verdrängungskammer (12, 13) umfasst, welche ein variables Volumen aufweist, wobei die Kammer eine Spritzenstruktur umfasst, die einen Kolben beinhaltet, der in einem Zylinder durch eine kraftbetriebene Einrichtung zum Variieren des Volumens der Verdrängungskammer beweglich ist.

13. Vorrichtung nach einem der vorhergehenden Ansprüche, wobei die Aufblaseinrichtung durch eine kraftbetriebene Einrichtung (14) angetrieben wird, die von einer Batterie (7) angetrieben wird.

## Revendications

1. Appareil (1) de traitement de troubles utérins par induction d'une nécrose d'un endomètre utérin, l'appareil comprenant :
• un cathéter (3) ayant une extrémité proximale et une extrémité distale ;
• une vessie (2) fixée à l'extrémité proximale pour son insertion dans l'utérus et pouvant se distendre sous l'effet de l'introduction d'un milieu de gonflage ;
• une chambre de stockage (13) contenant une quantité du milieu de gonflage,
• un moyen de gonflage (12, 13, 14) raccordé à ladite extrémité distale et étant fonctionnel dans une direction positive pour introduire le milieu de gonflage de cette chambre de stockage dans la vessie et ainsi distendre la vessie dans une direction négative pour retirer le milieu de gonflage de la vessie et ainsi dégonfler la vessie ; et
• un moyen de chauffage (15) configuré pour chauffer le milieu de gonflage dans la chambre de stockage avant l'insertion de la vessie dans le corps d'un sujet à une température suffisante pour induire une nécrose du tissu ;
dans lequel l'appareil est configuré pour l'actionnement simultané du moyen de chauffage et du moyen de gonflage et dans lequel le moyen de gonflage est configuré pour contrebalancer un changement de pression dans la vessie provoqué par l'expansion thermique du milieu de gonflage durant le chauffage dans la chambre de stockage,
comprenant en outre un moyen de commande électronique (6) communiquant avec le moyen de gonflage (12, 13, 14) et un moyen de détection de pression pour réguler le gonflage, et
dans lequel le moyen de commande est configuré pour fournir un signal électrique pour actionner le moyen de gonflage pour contrebalancer un changement de pression dans la vessie provoqué par l'expansion thermique du milieu de gonflage durant le chauffage dans la chambre de stockage avant l'insertion de la vessie dans le corps d'un sujet.

2. Appareil selon la revendication 1, comprenant en outre un moyen de détection de pression (17) agencé pour détecter une pression du milieu de gonflage, et dans lequel le moyen de commande est configuré pour actionner le moyen de gonflage dans la direction négative en réponse à une augmentation détectée de pression du milieu de gonflage.

3. Appareil selon l'une quelconque des revendications précédentes, comprenant en outre un moyen de détection de température (18) agencé pour détecter la température du milieu de gonflage, et dans lequel le moyen de commande est configuré pour actionner le moyen de gonflage dans la direction négative en réponse à une augmentation détectée de température.

4. Appareil selon l'une quelconque des revendications précédentes, dans lequel le moyen de commande est configuré pour générer un signal d'erreur si le moyen de gonflage n'est pas actionné dans la direction négative durant le chauffage.

5. Appareil selon l'une quelconque des revendications précédentes, dans lequel le moyen de commande est configuré pour générer un signal d'erreur si la pression du milieu de gonflage n'augmente pas durant le chauffage pour ainsi fournir une détection de fuite.

6. Appareil selon l'une quelconque des revendications précédentes, dans lequel le moyen de commande comprend un moyen mémoire, le moyen mémoire comprenant des informations spécifiant une séquence prédéfinie entraînant l'ablation thermique d'un patient, la séquence incluant l'étape consistant à contrebalancer le changement de pression provoqué par l'expansion thermique du milieu de gonflage durant le chauffage.

7. Appareil selon la revendication 6, dans lequel la séquence prédéfinie comprend les étapes suivantes :
a) actionnement du moyen de gonflage dans la direction positive pour gonfler la vessie ;
b) maintien de la pression dans la vessie durant la période de temps prédéfinie ;
c) actionnement du moyen de gonflage dans la direction négative pour dégonfler la vessie ; et
d) transmission d'un signal de fin indiquant le dégonflage de la vessie.

8. Appareil selon la revendication 7, comprenant un moyen de commande de moteur configuré pour déterminer une consommation d'énergie du moyen de gonflage et dans lequel le signal de fin est transmis en réponse à la détection d'une consommation d'énergie accrue.

9. Appareil selon l'une quelconque des revendications précédentes, dans lequel le moyen de gonflage pompe le milieu de gonflage de cette chambre de stockage dans la vessie.

10. Appareil selon la revendication 9, dans lequel la chambre de stockage contient une plus grande quantité du milieu de gonflage que ce qui est nécessaire pour gonfler la vessie.

11. Appareil selon la revendication 10, dans lequel le moyen de chauffage est configuré pour chauffer la totalité de la quantité du milieu de gonflage contenu dans la chambre de stockage et dans lequel le moyen de gonflage est adapté pour réaliser un dégonflage et un gonflage répétés de la vessie pour ainsi mélanger une partie du milieu de gonflage qui se trouve dans la vessie avec une partie du milieu de gonflage qui se trouve dans la chambre de stockage.

12. Appareil selon l'une quelconque des revendications précédentes, dans lequel le moyen de gonflage comprend une chambre de déplacement (12, 13) ayant un volume variable, la chambre comprend une structure de seringue comprenant un piston qui est mobile dans un cylindre par un moyen motorisé pour faire varier le volume de la chambre de déplacement.

13. Appareil selon l'une quelconque des revendications précédentes, dans lequel le moyen de gonflage est entraîné par un moyen mécanique (14) alimenté par une batterie (7).
